# EUROPÄISCHE PATENTSCHRIFT

(11) **EP 0 396 519 B1**
(45) Veröffentlichungstag und Bekanntmachung des Hinweises auf die Patenterteilung: **09.02.1994**
(21) Anmeldenummer: 90850146.3
(22) Anmeldetag: 20.04.1990
(51) Int. Cl.: A61F 2/42, A61F 2/30

(54) **Verankerungselement zum Halten eines Gelenkteils eines Fingers oder anderer künstlicher Gelenke**
Anchor element for holding a joint component of a finger or other artificial joints
Elément d'ancrage pour supporter une partie articulaire d'un doigt ou d'autres articulations artificielles

(30) Priorität: 25.04.1989 SE 8901508; 13.09.1989 US 406586
(43) Veröffentlichungstag der Anmeldung: 07.11.1990
(73) Patentinhaber: Branemark, Per-Ingvar, S-431 39 Mölndal (SE)
(72) Erfinder: Branemark, Per-Ingvar, S-431 39 Mölndal (SE)
(74) Vertreter: Barnieske, Hans Wolfgang

(56) Entgegenhaltungen:
- EP-A- 0 099 167
- WO-A-89/03663
- DE-A- 2 734 249
- DE-A- 2 808 740
- DE-A- 3 008 292
- DE-B- 2 338 136

## Beschreibung

### Hintergrund der Erfindung

### Gebiet der Erfindung

Die vorliegende Erfindung betrifft ein Verankerungselement zum Halten eines Gelenkteils und ein künstliches Gelenk.

Eine bevorzugte Ausführung der Erfindung wird im folgenden mit Bezug auf eine Wiederherstellung eines Fingergelenkes ausführlich beschrieben. Die Erfindung beschränkt sich jedoch nicht darauf. Die Erfindung kann für andre ähnliche Gelenke, wie für Zehengelenke, Ellbogengelenke u.s.w. verwendet werden. Die Erfindung kann auch bei Wiederherstellungen nach einer Amputation oder ähnlichen Verletzungen verwendet werden.

### Beschreibung verwandter Gebiete

Wiederherstellungen von Fingergelenken, die Prothesen enthalten, sind ursprünglich an MCP-Gelenken durchgeführt worden, die sich bei rheumatischer Erkrankung verändern. Prothesen, die für diesen Zweck bekannt sind, besitzen Swanson Silastic Fingergelenke. Solche Gelenke bestehen aus einem silikonähnlichen Material, sind länglich geformt, und haben Enden mit im wesentlichen kreisförmigen Querschnitt. Die Enden können in die Knochenmarkshöhlung jedes Knochens eingeführt und vorankert werden. Der Mittenteil der Prothese ist elastisch verformbar und bildet folglich das eigentliche Gelenkteil.

Die St. George-Prothese, eine verkittete Prothese, ist auch als künstliches Fingergelenk bekannt.

Ein Problem mit diesen konventionellen Prothesen besteht darin, daß sich das Verankerungselement, das den tatsächlichen Prothesenmechanismus hält, löst oder das umgebende Gewebe verletzt, und es zu einer ungewünschten Verschiebung der Prothese in Richtung der Belastung kommt.

Dazu wurden in den vergangenen Jahren Versuche unternommen, Halterungen aus Titan zu verwenden, die in der Knochenmarkshöhlung des Knochens verankert werden, mit der Absicht, daß diese verknöchernd einwachsen, wie es bei Hagert et al. beschrieben ist ("Metacarpophalangal Joint Replacement with Osseo-integrated Endoprostheses" in Scand. J. Plast. Reconstr. Surg. 20, Seiten 207 bis 218, 1986). Es ist bereits bekannt, orale und extraorale Prothesen im Knochengewebe zu verankern. Dieses verknöchernde Zusammenwachsen in der Zahntechnik ist in den letzen 25 Jahren von Prof. Brånemark und seinen Kollegen mit ausgezeichneten Ergebnissen beim Einsetzen von Halterungen in den Backenknochen, um Zähne oder Brükken zu halten, weiterentwickelt worden. Die Experimente, die von Hagert durchgeführt worden sind, um diese Technik auf die Wiederherstellung von Fingergelenken zu übertragen, haben die Erwartungen nicht erfüllt. Die unakzeptablen Ergebnisse sind offenbar auf die vollkommen unterschiedlichen Bedingungen zurückzuführen, die dann auftreten, wenn diese "Technik der Zahnmedizin" bei der Wiederherstellung von Fingergelenken mit einer Prothese angewandt wird. Zum Beispiel wird bei den bekannten Techniken die Halterung im rechten Winkel zur Längsachse des Knochens verankert. Im Fingergelenk wird die Halterung entlang der Achse des Knochens angeordnet. Demzufolge wirken auf die Verankerungselemente völlig verschiedene Belastungen und Kräfte.

Heute besteht das hauptsächliche Problem in der orthopädischen Chirurgie mit Prothesen nach wie vor in der Lockerung der Knochenverankerungseinheit. Jedoch treten mit einer Erfolgsrate für die zahntechnischen Implantate von mehr als 90 % über einen Zeitraum von 20 Jahren, eine Reihe anderer Probleme auf, die insoweit bisher nicht in Betracht gezogen werden mußten. Eines der hauptsächlichen Probleme ist eine zunehmende Abnutzung des Gelenkteils. So ist eine andere Art der Ausführung von Prothesen im Vergleich zur bisher gebräuchlichen gefragt, wenn die verknöchernd zusammenwachsende Methode angewendet werden soll. Um das Gelenkteil austauschen zu können ohne die Knochenverankerung zu verletzen, muß das System der Prothesen in Komponenten aufgeteilt werden, in denen das Gelenkteil von dem tatsächlichen Verankerungselement abgetrennt werden kann. Darüber hinaus muß, wenn das zweistufige Verfahren verwendet wird, es möglich sein, das Gelenkteil in einem zweiten Schritt zu verbinden, wenn der Patient oder zumindest das künstliche Gelenk des Patienten nicht unbeweglich werden soll. Dazu müssen zwei Umstände in Betracht gezogen werden: Erstens, das Gelenkteil unterliegt der Abnutzung und muß deshalb austauschbar sein. Zweitens, muß das Gelenkteil austauschbar sein, um ein zweistufige Verfahren zuzulassen.

Die Offenlegungschrift DE-A-3008292 gibt eine Mittelhand-Fingerknochenprothese an, deren Gelenkteil mittels Anschlusszapfen in die zylindrische axiale Bohrung jeweils eines kegelstumpfartigen buchsenförmigen Verankerungselements eingreift. Zumindestens einer der Anschlusszapfen läuft frei in der axialen Bohrung, wodurch eine Dehnbarkeit der Protese erzielt wird. Dies bekannten Verankerungselemente werden in den mit entsprechenden Bohrungen versehenen Knochen eingepresst und durch Oberflächenrauhigkeit, Rippen, u.ä. festgehalten; sie können auch durch Knochenzement fixiert werden.

Die gattungsgemäße Auslegeschrift DE-B-2338136 beschreibt ein zylinderbuchsenartiges, mit Aussengewinde zum Einschrauben in einen Knochen versehenes Verankerungselement zum Halten eines Gelenkteils. Die zwei Enden dieses Elements werden zweckmässigerweise mit Verbindungsende, d.h. dem Ende, an dem die Verbindung zwischen Verankerungselement und Gelenkteil hergestellt wird, und Einführungsende, d.h. dem Ende, das in eine Bohrung im Knochen zur Aufnahme des Verankerungselements eingeführt wird, bezeichnet. Das Einführungsende des bekannten Verankerungselements ist gegen den zylindrischen Hohlraum der Buchse hin offen. Das Verankerungselement weist von seinem Einführungsende zum Gelenkteil hin verlaufende Schlitze auf und besitzt eine Oberfläche, die wenigstens teilweise mit dem Gewebe des Knochens verknöchernd zusammenwachsen kann, um eine dauernde Verankerung im Inneren des Knochens in seiner Längsachse zu erreichen, wobei das Verankerungselement ein Material aufweist, das sich mit dem Knochengewebe verträgt.

### Zusammenfassung der Erfindung

Die vorliegende Erfindung betrifft eine Verbesserung derartiger bekannter buchsenartiger Verankerungselemente.

Die Erfindung basiert auf umfassenden experimentellen, biologischen Analysen des Aufbaus und der Funktion der Gelenke im Verlauf einer Krankheit oder während einer Abwehrreaktion nach einer Zersetzung des Knochengewebes durch Abnutzung oder Entzündung und umfassenden Studien der Blutzufuhr zum Knochenmark. Es wurde nachgewiesen, daß bei dem syntetischen Austausch von zerstörten, künstlichen Knorpeln und Sehnen, Knochen und Knochenmark als strukturelle und funktionelle Einheit behandelt werden müssen. Es ist unbedingt notwendig, diese für das Zusammenwachsen von Knochen und Knochenmark zu berücksichtigen, besonders bei langen Zeiträumen, wenn das harte Gewebe als Halt dienen muß.

Die Erfindung basiert folglich auf der Verwirklichung, daß bei der Verankerung eines prothetischen Austauches für eine Gelenkoberfläche und Sehnen zu Teilen des Knochengerüstes in Gelenknähe, die Zusammenwirkung zwischen Knochenmark und Knochengewebe berücksichtigt werden muß. Das bedeutet, daß ein Verknüpfungselement in der Knochenmarkshöhlung eines langen Knochens durch mechanische Elemente verankert werden muß, die einen Verbindungsweg zwischen Knochenmark und Knochengewebe so weit wie technisch möglich zulassen, möglichst über die gesamte Länge des Verankerungselementes wobei besonders auf die Grenzfläche zwischen Knochenmark und kompakten Knochen in der Wandung der Knochenmarkhöhlung Rücksicht genommen wird.

Ein gemäss diesen Voraussetzungen, insbesonders gemäss DE-B-23338136, gebildetes zylinderbuchsenförmiges Verankerungselement ist dadurch gekennzeichnet, dass die Dicke seiner Wandung allmählich gegen das Einführungsende hin abnimmt und die Wandung flexibel verformbar ist, wobei sich das Verankerungselement an das umgebende Knochengewebe anpasst, und dass die Schlitze Schneidkanten aufweisen.

Die Erfindung betrifft ausserdem ein künstliches Gelenk mit einem oder zwei erfindungsgemässen Verankerungselementen.

Andere Eigenschaften und Vorteile der Erfindung werden mit der folgenden Beschreibung einer bevorzugten Ausführung der Erfindung deutlich, mit dem Hinweis auf die begleitenden Zeichnungen.

### Kurze Beschreibung der Zeichnungen

- Fig. 1: ist ausschnittsweise eine Schnittansicht durch ein künstliches Fingergelenk mit einem Verankerungselement nach der Erfindung;
- Fig. 2: zeigt ein vollständiges künstliches Fingergelenk;
- Fig. 3: stellt dar, wie das Verankerungselement in dem langen Knochen angeordnet ist;
- Fig. 4: ist eine perspektivische Ansicht des Verankerungselmentes;
- Fig. 5: ist eine Schnittansicht des Gegenstandes der Fig. 4 entsprechend der Linie 5-5 in Fig. 4;
- Fig. 6: ist ausschnittsweise eine Schnittansicht durch den Gegenstand der Fig. 4 entsprechend der Linie 6-6 in Fig. 4, und stellt einen Teil des Gelenkteils nach der Erfindung dar;
- Fig. 7: varanschaulicht den Zusammenbau des Gelenkteils mit dem erfindungsgemässen Verankerungselement;
- Fig. 8: bis 10 veranschaulicht einige andere Ausführungsformen, die aufzeigen, wie eine nichtverdrehbare Verkeilung zwischen dem Verankerungselement und der Führungsbuchse erreicht und/oder verbessert werden kann.

### Ausführliche Beschreibung bevorzugte Ausführungsformen

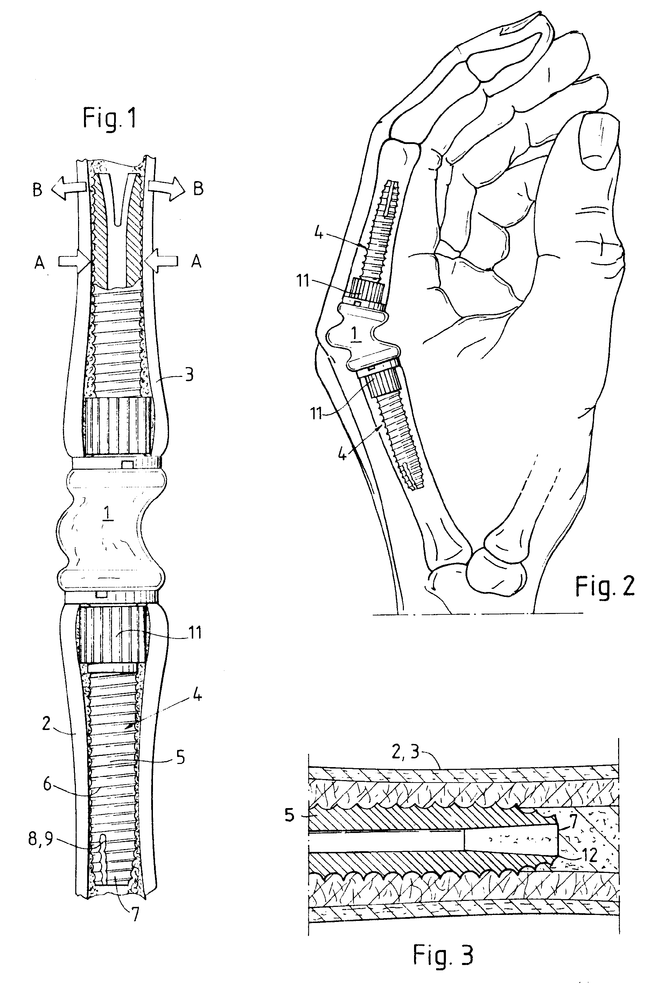

In Fig. 1 sind die Teile eines langen Knochens auf jeder Seite eines Gelenkteils 1 eines Fingers mit 2 und mit 3 bezeichnet.

Ein rotationssymmetrisches Verankerungselement 4 ist als hohler, im wesentlichen zylinderbuchsenartiger Körper 5 geformt, mit äußerem Gewinde 6 und abnehmender Dicke der Wandung. Das offene Einführungsende 7 des Körpers 5 ist konisch verjüngt und ist mit Schlitzen 8 und 9 versehen, die am offenen Einführungsende 7 beginnen.

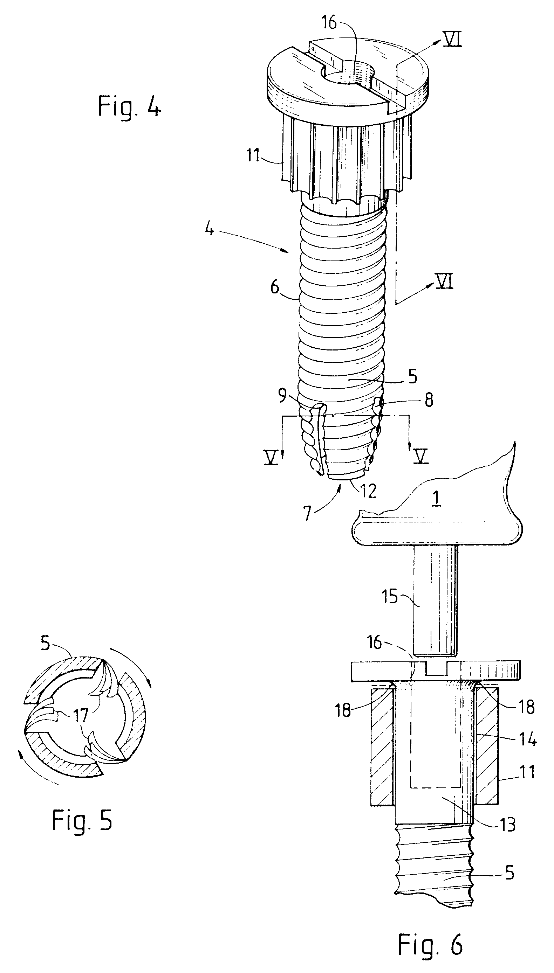

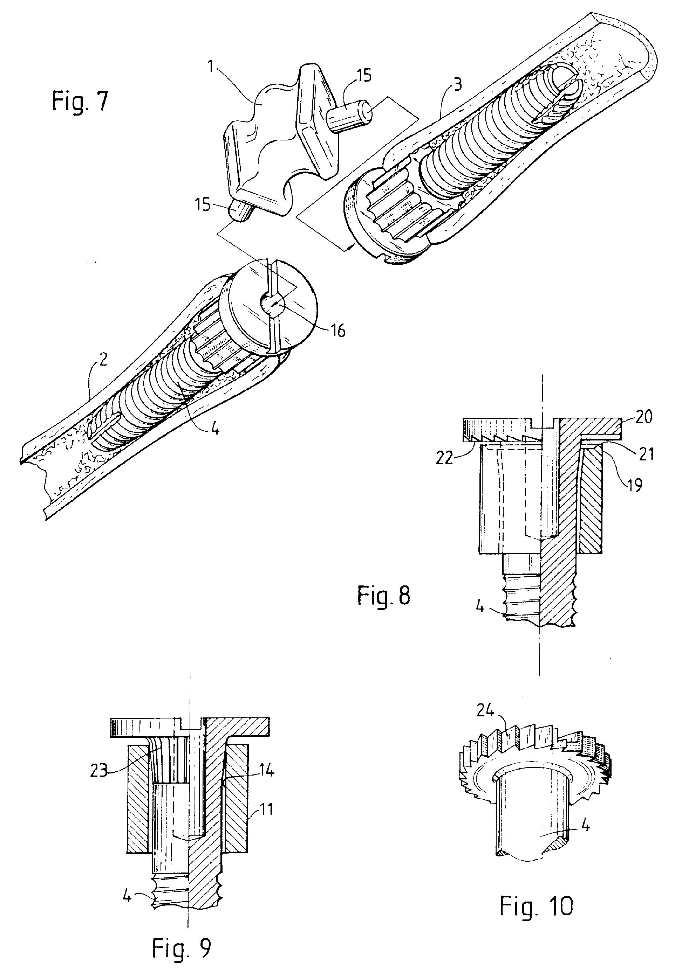

Ein Verbindungsende 13 (Fig. 6) des Verankerungselementes 4, das am nächsten zum Gelenkteil 1 liegt, ist in einer Führungsbuchse 11 angeordnet. Die gezeigte Führungsbuchse 11 ist im wesentlichen zylindrisch und ist an ihrer radialen äußeren Oberfläche mit einer längsverlaufenden Riffelung versehen. Diese Riffelung kann zweckmäßigerweise identisch sein mit einem axialen Abschnitt von konisch verjüngten, rotationssymmetrischen Halterungen, die in der Zahnheikunde verwendet werden. Die Führungsbuchse 13 umgibt das Verbindungsende 13. Das Verbindungsende 13 des Körpers 5 ist folglich durch eine Verkeilung mit der Innenfläche 14 der Führungsbuchse 11 dann fest verbunden, wenn der Körper 5 genügend weit in das Knochengewebe eingeschraubt ist. Das Verbindungsende 13 erhält ansteckbar Anschlußteile 15 des Gelenkteils 1. Die Anschlußteile 15 können zentrische Zapfen sein (Fig. 7), die aus dem Gelenkteil 1 herausragen. Solche Zapfen wirken mit entsprechenden Ausnehmungen 16 in dem Verankerungselement 4 oder in einem Verbindungsstück (nicht dargestellt), das zweckmäßigerweise zwischen der Führungsbuchse 11 und dem Gelenkteil 1 angeordnet ist, zusammen.

Ein oder mehrere Löcher können direkt mit der radialen äußeren Oberfläche des Verankerungselementes 4 verbunden sein, und zwar mit den Kanten der Löcher zu den oberflächenformenden Schneidkanten hin. Selbstschneidende Wirkung wird folglich erreicht, indem der Körper 5 in den Knochen 2 geschraubt wird. Das entfernte Knochengewebe 17 wird innerhalb des Verankerungselementes 4 aufgenommen, wie es Fig. 5 veranschaulicht.

Dort wo die Dicke der Wandung des Verankerungselementes 4 allmählich zum Einführungsende 7 abnimmt und/oder dort wo das Verankerungselement 4 die längsverlaufenden Schlitze 8 und 9 hat, ist die Anforderung nach einer guten Verformbarkeit erfüllt, was die Gefahr von Spannungskonzentrationen, die zu besonderen Problemen bei den bekannten Ausführungen dieser Art führt, erheblich reduziert. Die Pfeile B-B in Fig. 1 zeigen die Flexibilität des offenen Einführungsendes 7 auf, wie zum Beispiel seine Fähigkeit, sich an das umgebende Knochengewebe anzupassen. Das gleiche gilt für die Pfeile A-A.

Die Schlitze 8 und 9 können zweckmäßigerweise mit Schneidkanten versehen sein und, wenn die Aushöhlung das abgeschabte Knochengewebe 17 aufnimmt, wie es in Fig. 5 gezeigt ist, ist das Verankerungselement 4, in sich selbst, sein eigenes Vorbereitungswerkzeug. Gleichzeitig werden optimale Bedingungen für normale anatomische und physiologische Gegebenheiten sichergestellt, indem eine Beeinträchtigung des verbleibenden biologischen Gewebes, zum Beispiel des Knochenmarks und des Knochengewebes, minimiert wird. Fig. 2 zeigt ein künstliches Gelenk, wie es mit dem eingesetzten Verankerungselement erreicht wird.

Fig. 3 zeigt auf, wie das Verankerungselement in den Knochen 2 positioniert ist. In der Praxis ist das Verankerungselement 4 in der Grenzfläche zwischen Knochenmark und Knochengewebe positioniert. Die Wanddicke des zylinderbuchsenartigen Körpers 5 nimmt zum geschlitzten Einführungsende 7 hin ab und endet in einer konisch verjüngten Schneidkante 12. Die Kanten der längsverlaufenden Schlitze 8 können ebenso als Schneidkanten ausgeformt sein. Das abgeschabte Knochengewebe 17, das beim Einschrauben des Verankerungselementes 4 in die Knochenmarkhöhlung entsteht, wird in den Hohlraum innerhalb des Körpers 5 abgegeben, wie es Fig. 5 veranschaulicht.

Bei einer Operation wird ein Teil des langen Knochens 2 in der Nähe des Gelenkes abgeschnitten, so daß die Knochenmarkhöhlung freigelegt wird. Dann wird eine Sonde (nicht dargestellt) in die Knochenmarkhöhlung eingeführt, um eine verwendbare Längsachse für das Einführen des Verankerungselementes 4 bestimmen zu können. Die Sonde wird zur Richtungsfindung eingesetzt, um das Verankerungselement 4 wie gewünscht zentrisch ansetzen zu können. Dann wird in die freiliegende Knochenmarkhöhlung sorgfältig ein Ansatz für die Führungsbuchse 11 gebohrt. Nachdem die Führungsbuchse eingesetzt worden ist, wird der zylinderbuchsenartige Körper 5 in die Knochenmarkhöhlung, durch die Führungsbuchse 5 hindurch, eingeschraubt. Die Sonde ist selbstverständlich vorher entfernt worden. Das Verbindungsende 13 des Verankerungselementes 4 außen größerwerdend aufgeweitet, wie es bei 18 dargestellt ist, so daß sein Durchmesser geringfügig größer ist als die darunterliegende Öffnung der Führungsbuchse 11. So ergibt sich schließlich eine Verkeilung zwischen dem Verbindungsende 13 und der Führungsbuchse 11 (wenn die Führungsbuchse 11 in die durch gestrichelte Linien in Fig. 6 angedeutete Position gebracht wird), so daß beide, Führungsbuchse 11 und zylinderbuchsenartiger Körper 5, in die gewünschte Lage gebracht sind.

Das Verankerungselement 4 und die Führungsbuchse 11 bestehen oder sind hergestellt aus Titan oder sind mit Titan beschichtet. Die Struktur der Titanoberfläche ist so ausgebildet, daß wenigstens an den wichtigen Teilen der Oberfläche ein Zusammenwachsen gefördert wird. Vorteilhafterweise kann die Oberfläche (wie es beispielsweise in der schwedischen Patentschrift SE 7902035-0 beschrieben ist) Ungleichmäßigkeiten in Form von Mikrodellen aufweisen mit einem Dellendurchmesser zwischen 10 und 1000 nm, vorzugsweise 10 bis 300 nm. Alternativ dazu können der Titanoberflächenschicht chemische Bestandteile zugegeben werden, die eine besondere positive Gewebereaktion bewirken können,
wie es zum Beispiel in der schwedischen Patentschrift SE 8505158-9 beschrieben wird.

Fig. 8 zeigt, wie das obere Ende 19 der Führungsbuchse 11, das dem Flansch 20 des Verankerungselementes 4 zugekehrt ist, mit einer vorkragenden Kante 21 versehen ist, die angebracht ist, um mit Verzahnungen 22 des Flansches 20 zusammenzuwirken. Das Verankerungselement 4 und die Führungsbuchse 11 sind folglich nicht verdrehbar und fest verbunden, wenn der Körper 5 weit genug in die Knochensubstanz eingeschraubt ist.

Eine alternative Verbindungsmöglichkeit zwischen der Führungsbuchse 11 und dem Verankerungselement 4 wird mit der Anordnung, wie sie in Fig. 9 gezeigt ist, erreicht, wobei eine axiale Riffelung angebracht ist, die mit der Innenfläche der Führungsbuchse 11 zusammenwirkt.

Eine weitere Verbindungsmöglichkeit kann durch die Anordnung, die in Fig. 10 gezeigt wird, erreicht werden, indem der obere Außenrand des Verankerungselementes 4, der in das Knochengewebe eingeschraubt wird, eine spezielle Verzahnung erhält.

Obwohl die Erfindung in Verbindung mit bevorzugten Ausführungsformen beschrieben worden ist, werden dem Fachmann hierüberhinaus viele andere Abwandlungen wie auch Modifikationen und andere Einsatzmöglichkeiten eröffnet.

## Patentansprüche

1. Zylinderbuchsenartiges, mit Aussengewinde zum Einschrauben in einen Knochen (2) versehenes Verankerungselement (4) zum Halten eines Gelenkteils (1) an seinem Verbindungsende (13), wobei das Verankerungselement (4) wenigstens teilweise hahl ist und von seinem offenen Einführungsende (7) zum Gelenkteil (1) hin verlaufende Schlitze (7, 8) aufweist und eine Oberfläche besitzt, die wenigstens teilweise mit dem Gewebe des Knochens (2) verknöchernd zusammenwachsen kann um eine dauerhafte Verankerung im Inneren des Knochens entlang seiner Längsachse zu erreichen, wobei das Verankerungselement (4) ein Material aufweist, das sich mit dem Knochengewebe verträgt, dadurch gekennzeichnet, dass die Dicke der Wandung des Verankerungselements (4) allmählich gegen das Einführungsende (7) hin abnimmt und die Wandung flexibel verformbar ist, wobei sich das Verankerungselement (4) an das umgebende Knochengewebe anpasst, und dass die Schlitze (8, 9) Schneidkanten aufweisen.

2. Verankerungselement nach Anspruch 1. dadurch gekennzeichnet, dass es eine das Verbindungsende (13) umgebende Führungsbuchse (11) aufweist, wobei das Verbindungsende (13) in der Führungsbuchse (11) verdrehsicher fixierbar ist.

3. Verankerungselement nach Anspruch 1 oder 2, dadurch gekennzeichnet, dass das Verbindungsende (13) zur ansteckbaren Aufnahme von Anschlussteilen (15) des Gelenkteils (1) ausgebildet ist.

4. Verankerungselement nach Anspruch 3, dadurch gekennzeichnet, dass zwischen dem Gelenkteil (1) und der Führungsbuchse (11) ein Verbindungsstück angeordnet ist.

5. Verankerungselement nach einem der vorhergehenden Ansprüche, dadurch gekennzeichnet, dass es aus Titan besteht.

6. Verankerungselement nach einem der Ansprüche 1 bis 4, dadurch gekennzeichnet, dass seine Oberfläche mit Titan beschichtet ist.

7. Verankerungselement nach einem der Ansprüche 1 bis 4, dadurch gekennzeichnet, dass seine Oberfläche eine radiale äussere Oberfläche ist und wenigstens teilweise Mikrodellen mit einem Durchmesser von 10 bis 300 nm hat.

8. Verankerungselement nach einem der vorhergehenden Ansprüche, dadurch gekennzeichnet, dass das Gelenkteil (1) ein Teil eines künstlichen Fingergelenks ist.

9. Künstliches Gelenk mit einem oder zwei Verankerungselementen (4) nach Anspruch 1 bis 8.

## Claims

1. An anchoring element (4) in the form of a cylindrical bush and having an external screwthread for screwing into a bone (2) in order to hold a joint member (1) at its connecting end (13), the anchoring element (4) being at least partially hollow and having slots (7, 8) extending from its open introduction end (7) to the joint member (1), and having a surface which can at least partially take to the tissue of the bone (2) in order to give a permanent anchorage in the interior of the bone along its longitudinal axis, the anchoring element (4) comprising a material which is compatible with the bone tissue, characterised in that the thickness of the wall of the anchoring element (4) gradually decreases towards the introduction end (7) and the wall is flexibly deformable, the anchoring element (4) adapting to the surrounding bone tissue, and in that the slots (8, 9) have cutting edges.

2. An anchoring element according to claim 1, characterised in that it comprises a guide bush (11) surrounding the connecting end (13), the latter being fixable in the guide bush (11) so as to be secured against turning.

3. An anchoring element according to claim 1 or 2, characterised in that the connecting end (13) is so constructed as to receive pushed-on junction members (15) of the joint member (1).

4. An anchoring element according to claim 3, characterised in that there is a connector between the joint member (1) and the guide bush (11).

5. An anchoring element according to any one of the preceding claims, characterised in that it consists of titanium.

6. An anchoring element according to any one of claims 1 to 4, characterised in that its surface is coated with titanium.

7. An anchoring element according to any one of claims 1 to 4, characterised in that its surface is a radial outer surface and has at least partially micro-cavities having a diameter of 10 to 300 nm.

8. An anchoring element according to any one of the preceding claims, characterised in that the joint member (1) is part of an artificial finger joint.

9. An artificial joint having one or two anchoring elements (4) according to claims 1 to 8.

## Revendications

1. Elément d'ancrage (4), de forme cylindrique, muni d'un filetage extérieur permettant un vissage dans un os (2), pour soutenir une pièce articulée (1) en son extrémité d'assemblage (13), l'élément d'ancrage (4) étant au moins partiellement creux et comportant des entailles (8, 9), qui s'étendent de son extrémité d'introduction ouverte (7) jusqu'à la pièce articulée (1), et présentent une surface qui, au moins partiellement, peut croître avec ossification en même temps que le tissu de l'os (2), de façon à assurer un ancrage permanent à l'intérieur de l'os le long de son axe longitudinal, l'élément d'ancrage (4) étant en un matériau compatible avec le tissu osseux, caractérisé en ce que l'épaisseur de la paroi de l'élément d'ancrage (4) diminue progressivement vers l'extrémité d'introduction (7), et que la paroi peut subir une déformation souple, l'élément d'ancrage (4) étant adapté au tissu osseux environnant, et en ce que les entailles (8, 9) présentent des arêtes coupantes.

2. Elément d'ancrage selon la revendication 1, caractérisé en ce qu'il présente une douille de guidage (11), qui entoure l'extrémité d'assemblage (13), l'extrémité d'assemblage (13) étant fixée dans la douille de guidage (11) de façon à ne pouvoir subir de torsion.

3. Elément d'ancrage selon la revendication 1 ou 2, caractérisé en ce que l'extrémité d'assemblage (13) est conçue pour recevoir, par emboitement, des éléments de raccordement (15) de la pièce articulée (1).

4. Elément d'ancrage selon la revendication 3, caractérisé en ce qu'une pièce d'assemblage est disposée entre la pièce articulée (1) et la douille de guidage (11).

5. Elément d'ancrage selon l'une des revendications précédentes, caractérisé en ce qu'il est en titane.

6. Elément d'ancrage selon l'une des revendications 1 à 4, caractérisé en ce que sa surface est revêtue de titane.

7. Elément d'ancrage selon l'une des revendications 1 à 4, caractérisé en ce que sa surface est une surface radiale extérieure et présente au moins partiellement des microdéformations ayant un diamètre de 10 à 300 nm.

8. Elément d'ancrage selon l'une des revendications précédentes, caractérisé en ce que la pièce articulée (1) est une partie d'une articulation digitale artificielle.

9. Articulation artificielle comportant un ou deux éléments d'ancrage selon les revendications 1 à 8.
